**Europäisches Patentamt**

**European Patent Office**

**Office européen des brevets**

(11) Veröffentlichungsnummer: **0 144 005**
**B1**

# EUROPÄISCHE PATENTSCHRIFT

(45) Veröffentlichungstag der Patentschrift:
**26.08.87**

(21) Anmeldenummer: **84113292.1**

(22) Anmeldetag: **05.11.84**

(51) Int. Cl.⁴: **A 61 B 17/16**, B 25 D 9/20,
B 25 D 9/26

(54) **Pneumatisches Schlagwerkzeug.**

(30) Priorität: **01.12.83 CH 6428/83**

(43) Veröffentlichungstag der Anmeldung:
**12.06.85 Patentblatt 85/24**

(45) Bekanntmachung des Hinweises auf die Patenterteilung:
**26.08.87 Patentblatt 87/35**

(84) Benannte Vertragsstaaten:
**AT BE DE FR GB IT LU NL SE**

(56) Entgegenhaltungen:
**FR - A - 1 348 165**
**GB - A - 2 014 651**
**US - A - 2 655 921**
**US - A - 2 725 878**

(73) Patentinhaber: **Emil Schenker AG, Stauwehrstrasse 34,
CH-5012 Schönenwerd (CH)**

(72) Erfinder: **Karpf, Kurt, Alte Strasse 175,
CH-4718 Holderbank (CH)**
Erfinder: **Bobst, Franz, Kirchgasse, CH-4702 Oensingen
(CH)**

(74) Vertreter: **Fillinger, Peter, Dr., Rütistrasse 1a,
CH-5400 Baden (CH)**

ACTORUM AG

## Beschreibung

Die vorliegende Erfindung bezieht sich auf ein pneumatisches Schlagwerkzeug mit einer mit einem pneumatischen Zylinder fest verbundenen Fassung zur Aufnahme eines Bearbeitungswerkzeuges und mit einem als Hammer wirkenden Kolben, der auf einer ersten Seite für den gegen das Bearbeitungswerkzeug gerichteten Kolbenvorlauf eine intermittierend mit Druck beaufschlagbare erste Fläche aufweist und dessen andere Seite als gegen das Bearbeitungswerkzeug wirkende Hammerflächen ausgebildet ist.

Beim Einsetzen künstlicher Gelenke in einen lebenden Organismus wird meistens die Knochenmarkhöhle geöffnet, um darin den künstlichen Gelenkteil zu verankern. Die feste Verbindung zwischen dem Knochen und dem in den geöffneten Markraum hineinragenden Anker des Gelenkteils erfolgt durch Knochenzement, der den Zwischenraum zwischen dem Anker und dem Knochen ausfüllt. Es ist indessen auch bekannt, den Gelenkteilanker als stumpfer Keil mit kleinem Keilwinkel auszubilden und in der Knochenmarkhöhle Lagerflächen für die Keilflächen mit entsprechend kleinem Anzug spanabhebend herauszuarbeiten. In diesem Falle wird das Gelenkteil mit Reibschluss im Knochen zementlos verankert. Das Herausarbeiten der Lagerflächen in der Markhöhle erfolgt mittels Profilraspeln unterschiedlicher Breite. Dabei beginnt der Operateur mit der Raspel kleinster Breite und setzt den Bearbeitungsvorgang bis zur präoperativ, anhand des Röntgenbildes vorbestimmten Grösse fort, welche die Lagerfläche masslich genau in Übereinstimmung mit den Keilflanken am Gelenkteilanker bringt. Die Lagerflächen werden aus dem harten, corticalen Röhrenknochen herausgearbeitet, nachdem bereits der relativ weiche Spongiosaknochen entfernt wurde.

Für das Raspeln wird jeweils eine Raspel mit einem sogenannten Gleithammer von Hand in den Knochen hineingetrieben. Der Gleithammer weist am Ende einer Gleitstange eine Raspelfassung auf. Auf der Gleitstange ist ein Gewichtskörper verschiebbar gelagert, der durch den Operateur gegen eine Schlagfläche der Werkzeugfassung geschlagen wird.

Der manuelle Vortrieb der Raspel schafft durch seine Ungenauigkeit im Markraum ausreichend Spiel, um das Raspelwerkzeug wieder aus den Knochen herausziehen zu können. Der Nachteil dieses Hammers besteht, nebst dem unerwünschten Spiel zwischen Keil und Lagerflächen, darin, dass er wenig wirksam ist und für die Preparierung einer einzigen Protheseauflage im Oberschenkelknochen bis zu mehreren hundert Einzelschlägen erfordert, was zeitraubend ist und den Operateur ermüdet. Zudem werden die einzelnen Schläge auf den Patienten übertragen.

Weiter sind in der Technik Schlagwerzeuge zum Meisseln und Hämmern bekannt. Werden die erwähnten Raspeln mit solchen Geräten in der Markhöhle eines Knochens getrieben, lassen sich die Raspeln nach erfolgtem Eintrieb nicht mehr oder nur schwer aus der Markhöhle entfernen, so dass diese Schlagwerkzeuge nicht anstelle des erwähnten Gleithammers verwendbar sind.

Die vorliegende Erfindung stellt sich die Aufgage, ein Schlagwerkzeug bekannter Art derart zu verbessern, dass es die erwähnten Bearbeitungswerkzeuge nicht nur genau vortreibt, sondern auch, dass diese Werkzeuge jederzeit während des Vortriebs leicht aus der Knochenmarkhöhle herausgezogen werden können.

Erfindungsgemäss wird diese Aufgabe dadurch gelöst, dass am Kolben eine der erwähnten ersten Fläche entgegengerichtete, kleinere, für den Kolbenrücklauf intermittierend mit Druckluft beaufschlagbare zweite Fläche vorhanden ist und dass auf der erwähnten ersten Seite eine zweite Hammerfläche vorhanden ist, die am Ende des Kolbenrücklaufs auf die zugewandte Zylinderfläche auftrifft.

Durch die Erfindung wird die in die Markhöhle eintreibende Raspel nach jedem vortreibenden Schlag mit einem lockeren Rückschlag beaufschlagt, so dass sie in keiner Phase des Vortriebs form- und/oder materialschlüssig in der Markhöhle festgehalten ist. Der Knochen wird schonender behandelt und gleichzeitig können die Keilflächen wesentlich genauer herausgearbeitet werden. Zudem werden die entstehenden Knochenspäne besser aus der Markhöhle ausgetragen.

Anhand der beiliegenden schematischen Zeichnung wird die Erfindung beispielsweise erläutert. Es zeigen:

Fig. 1 einen Längsschnitt durch ein Schlagwerkzeug, wobei sich der Kolben in der vorderen Endlage befindet, in der er einen Schlag auf die Raspel ausübt,

Fig. 2 eine gleiche Darstellung wie Fig. 1, wobei der Kolben im Rücklauf mittlere Stellung einnimmt,

Fig. 3 eine gleiche Darstellung wie Fig. 1, wobei der Kolben die hintere Endlage einnimmt und

Fig. 4 eine gleiche Darstellung wie Fig. 1, wobei der Kolben im Vorlauf eine mittlere Stellung einnimmt.

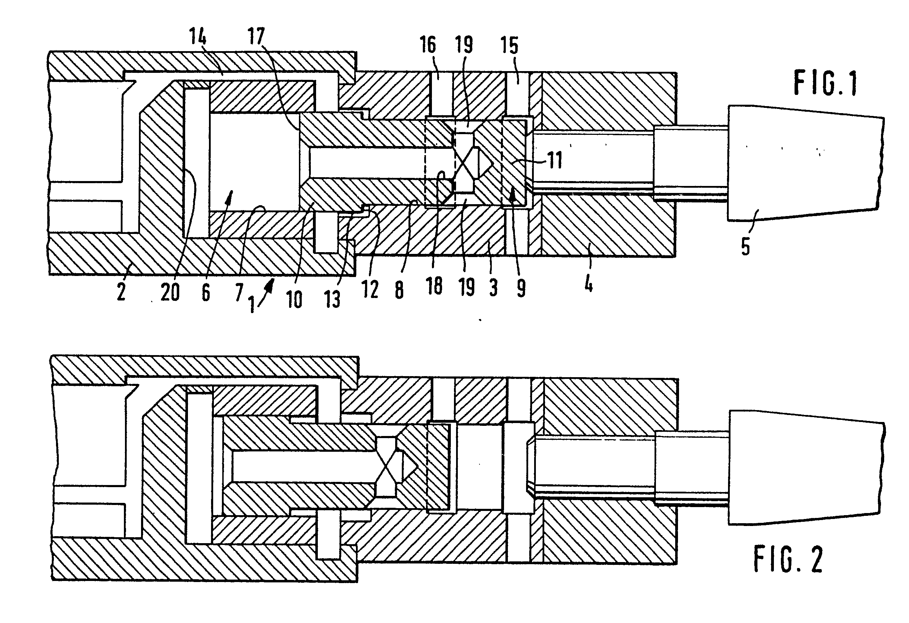

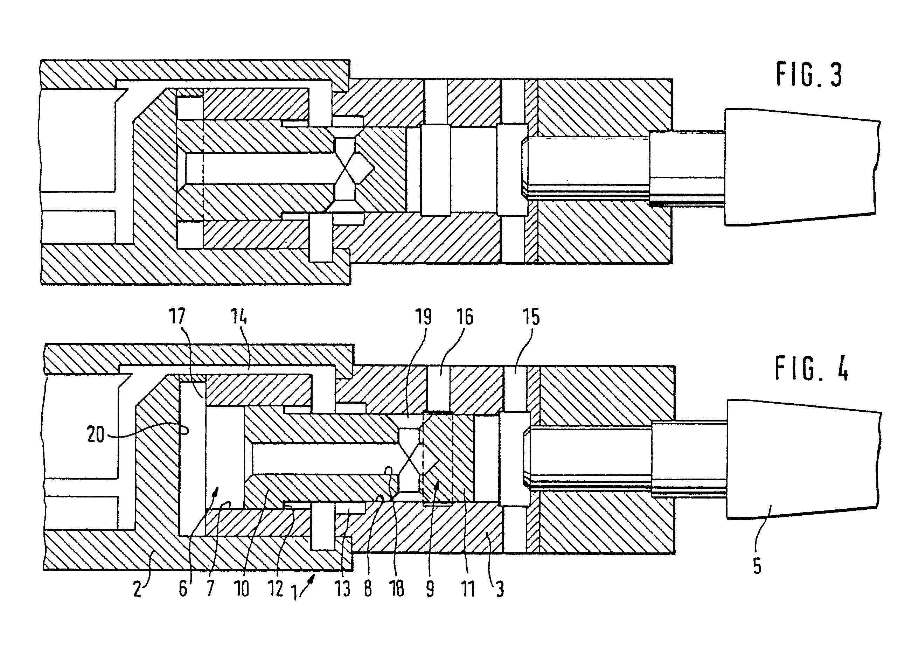

Das in der Zeichnung gezeigte Schlagwerkzeug weist einen pneumatischen Zylinder 1 bestehend aus den axial ineinandergesteckten Teilen 2 und 3 auf, wobei das vordere Ende des Teils 3 fest mit einer Werkzeugfassung 4 für eine Raspel 5 verbunden ist. Der Zylinder 1 bildet einen axial an die Raspel 5 anschliessenden Zylinderraum 6, der einen Abschnitt 7 mit grösserem und einen Abschnitt 8 mit kleinerem Durchmesser aufweist. Im Zylinderraum 6 ist axial verschiebbar ein Kolben 9 gelagert, der abgestuft entsprechend dem Zylinderraum, zwischen einer vorderen (in Fig. 1 gezeigten) in einer hinteren (in Fig. 3 gezeigten) Endlage verschiebbar ist. Der Kolben 9 weist entsprechend dem Zylinderraum 6 einen Abschnitt 10 grösseren und einen Abschnitt 11 kleineren Durchmessers auf, wodurch eine Schulterfläche 12 den Übergang bildet. Befindet sich der Kolben 9 in der in Fig. 1 gezeigten vorderen Endlage, so ist der Bereich der Schulterfläche 12 von einer Ringkammer 13 grösseren Durchmessers umgeben, in die eine Luftzuführleitung 14 mündet. Am raspelseitigen Ende ist der Zylinderraum 6 durch einen mit der Aussenatmosphäre verbundenen Kanal 15 dauernd entlüftet. Zwischen dem Kanal 15 und der Ringkammer 16 mündet in den Zylinderabschnitt 8 ein zweiter Entlüftungskanal 16.

Der Kolben 9 weist rückseitig eine mit Druckluft beaufschlagbare Fläche 17 auf, welche zusammen mit den übrigen, der Raspel 5 abgewandten Kolbenflächen wesentlich grösser als die Schulterfläche 12 ist. In dieser Fläche 17 mündet eine zentrale Sackbohrung 18 von der Verzweigungen 19 in die Kolbenmantelfläche münden. In der vorderen, in Fig. 1 gezeigten Endlage des Kolbens 9 sind durch die Mündung 19 die Sackbohrungen 18 und der angrenzende Teil der Zylinderkammer 6 entlüftet, wogegen der in der Luftzuführleitung 14 herrschende Druck über die Ringkammer 13 auf die Schulterfläche 12 wirkt. Hierdurch wird der Kolben 9 nach rückwärts getrieben. Durch das schlagartige Auftreffen des Kolbens 9 (am Ende des Vorlaufs) auf die Raspel 5 erfährt er einen elastischen Rückschlag, wobei diese Bewegung durch den auf die Schulterfläche 12 wirkenden Druck unterstützt wird. Sobald die Verzweigung 19 in den Bereich der Ringkammer 13 gelangt (Fig. 2) strömt Druckluft in die Sackbohrung 18 und den anschliessenden Teil der Zylinderkammer, wodurch der Rücklauf des Kolbens 9 gebremst wird. Die Bremswirkung soll indessen (bedingt durch ein entsprechendes Verhältnis der einander entgegengerichtet wirksamen Kolbenfläche 12 und 17) so bemessen sein, dass der Kolben 9 mit der Fläche 17 auf die Rückschlagfläche 20 aufschlägt und der Raspel 5 einen dem Vortrieb entgegengesetzt gerichteten elastischen Rückschlag induziert. Der sich in der Sackbohrung 18 aufbauende Luftdruck (Fig. 3) lässt den Kolben 9 wieder gegen die Raspel 5 vorlaufen, wobei die Vortriebskraft zusammenfällt, sobald die Verzweigung 19 den Kanal 16 erreicht und die vortreibende Druckluft abgeblasen wird. Der vorlaufende Kolben 9 trifft wieder schlagartig auf die Raspel 5 auf und treibt diese weiter in die Markhöhle 5 eines Knochens vor. Der elastische Rückschlag und der auf die Schulterfläche 12 wirkende Luftdruck schleudern den Kolben 9 wieder zurück, bis die Fläche 17 gegen die Rückschlagfläche 20 aufschlägt. Der Kolbenrücklauf erfährt gegen sein Ende eine Bremsung, wenn erneut Druckluft auf der Ringkammer 13 in die Verzweigung 19 strömt. Je grösser die Schulterfläche 12 gewählt wird (wobei der Durchmesser des Kolbenabschnittes 11 entsprechend zu verkleinern wäre), umso schneller ist die Rücklaufbewegung des Kolbens 9 und umso grösser die Rückschlagkraft die er beim Auftreffen auf die Rückschlagfläche 20 erzeugt.

Nach einem nicht dargestellten Ausführungsbeispiel kann vorgesehen sein, dass die Rückschlagfläche 20 in axialer Richtung zum Kolben 9 verstellbar ausgeführt ist. Dadurch könnte der vom Kolben 9 erzeugte Rückschlag vergrössert oder verkleinert werden.

## Patentansprüche

1. Pneumatisches Schlagwerkzeug mit einer mit einem pneumatischen Zylinder (1) fest verbundenen Fassung (4) zur Aufnahme eines Bearbeitungswerkzeuges (5) und mit einem als Hammer wirkenden Kolben (9), der auf einer ersten Seite für den gegen das Bearbeitungswerkzeug gerichteten Kolbenvorlauf eine intermittierend mit Druck beaufschlagbare erste Fläche (17) aufweist und dessen andere Seite als gegen das Bearbeitungswerkzeug wirkende Hammerfläche ausgebildet ist, dadurch gekennzeichnet, dass am Kolben (9) eine der erwähnten ersten Fläche (17) entgegengerichtete, kleinere, für den Kolbenrücklauf intermittierend mit Druck beaufschlagbare zweite Fläche (12) vorhanden ist, und dass auf der erwähnten ersten Seite eine zweite Hammerfläche (17) vorhanden ist, die am Ende des Kolbenrücklaufs auf die zugewandte Zylinderfläche (20) auftrifft.

2. Schlagwerkzeug nach Anspruch 1, dadurch gekennzeichnet, dass der Kolben in axialer Richtung in zwei aneinandergrenzende Abschnitte (10, 11) unterteilt ist, dass der erste Abschnitt (10) die erste Fläche (17) und der zweite Abschnitt (11) die erste Hammerfläche und einen kleineren Durchmesser als der erste Abschnitt (11) aufweist, wobei der Übergang zwischen den beiden Abschnitten (10, 11) eine die zweite Fläche (12) bildende Schulter ist, und dass das Öffnen und Schliessen der in Zylinder mündenden Be- und Entlüftungskanäle (14, 15, 16) durch den Kolben (9) erfolgt.

3. Schlagwerkzeug nach Anspruch 2, dadurch gekennzeichnet, dass der Kolben (9) in der vorderen Endlage im Bereich der Schulterfläche (12) von einer Ringkammer (13) grösseren Durchmessers umgeben und die Ringkammer (13) mit Druckluft beaufschlagbar ist, dass in den anschliessenden Zylinderabschnitt (8) kleineren Durchmessers eine Entlüftungsleitung (15) mündet, dass je in die erste Kolbenseite (17) und in den Kolbenabschnitt (11) kleineren Durchmessers ein im Kolben angeordneter Luftkanal (18, 19) mündet, wobei die Mündung (19) im Kolbenabschnitt (11) kleineren Durchmessers in einer Endlage an eine durch den Zylinder geführte Entlüftungsleitung (16) und in der anderen Endlage an die Ringkammer (13) anschliesst.

## Claims

1. Pneumatic percussion tool, with a holder (4) for accommodating a processing tool (5), said holder being connected securely to a pneumatic cylinder (1), and with a piston (9) which acts as a hammer and which, at a first side, has a first space (17) adapted to be acted upon intermittently with pressure for the piston advance directed towards the processing tool, and whose other side is constructed as a hammer face acting against the processing tool, characterised in that there is provided on the piston (9) a smaller second face (12) which is directed oppositely to the said first face (17) and is adapted to be acted upon intermittently with compressed air for the return travel of the piston, and that there is provided at the said first side a second hammer face (17) which at the end of the return travel of the piston impinges against the facing cylinder surface (20).

2. Percussion tool according to claim 1, characterised in that the piston is sub-divided in the axial direction into two portions (10, 11) adjacent one another, that the first portion (10) has the first face (17) and the second portion (11) has the first hammer face and a smaller diameter than the first portion (11), the transition between the two portions (10, 11) being a shoulder forming the second face (12), and that the opening and closing of the air supply and venting ducts (14, 15, 16) opening into the cylinder is effected by the piston (9).

3. Percussion tool according to claim 2, characterised in that in the forward end position the piston (9) is surrounded in the region of the shoulder face (12) by an annular chamber (13) of relatively large diameter, and the annular chamber (13) is adapted to have compressed air supplied to it, that a venting duct (15) opens into the adjoining cylinder portion (8) which is relatively small in diameter, that air ducts (18, 19) arranged in the piston open one each into the first piston side (17) and into the piston portion (11) of relatively small diameter respectively, the mouth (19) in the piston portion (11) of relatively small diameter connecting in one end position with a venting duct (16) extending through the cylinder and connecting in the other end position with the annular chamber (13).

**Revendications**

1. Outil de percussion pneumatique comportant une monture (4) reliée rigidement à un vérin pneumatique (1) pour recevoir un outil d'usinage (5), et un piston (9) fonctionnant en marteau, lequel marteau comporte, sur une première face d'un premier côté une première surface (17) sur laquelle une pression peut être appliquée de façon intermittente pour la course du piston vers l'outil d'usinage, et dont l'autre côté est réalisée sous la forme d'une surface de marteau agissant contre l'outil d'usinage, caractérisé en ce que le piston (9) comporte une seconde surface (12) dirigée en sens opposé par rapport à ladite première surface (17), plus petite, sur laquelle peut être appliqué de façon intermittente de l'air comprimé pour la course de retour du piston, et en ce que ledit premier côté comporte une seconde surface de marteau (17) qui heurte, à la fin de la course de retour du piston, la surface (20) du vérin tournée vers elle.

2. Outil de percussion selon la revendication 1, caractérisé en ce que le piston est subdivisé, en direction axiale, en deux sections (10, 11) contiguës, en ce que la première section (10) comporte la première surface (17) et la seconde section (11) la première surface de marteau et présente un plus petit diamètre que la première section (11), la transition entre les deux sections (10, 11) étant un épaulement constituant la seconde surface (12), et en ce que l'ouverture et la fermeture des canaux d'amenée et d'évacuation d'air (14, 15, 16) débouchant dans le vérin sont effectuées par le piston (9).

3. Outil de percussion selon la revendication 2, caractérisé en ce que le piston (9) est entouré, dans la position terminale antérieure, dans la zone de la surface (12) de l'épaulement, par une chambre annulaire (13) de plus grand diamètre, la chambre annulaire (13) pouvant recevoir de l'air comprimé, en ce qu'une conduite d'évacuation d'air (15) débouche dans la section de cylindre (8) contiguë de plus petit diamètre, en ce qu'un canal d'aération (18, 19) placé dans le piston débouche respectivement dans le premier côté (17) du piston et dans la section de piston (11) de plus petit diamètre, l'embouchure (19) de la section de piston (11) de plus petit diamètre donnant, dans une position terminale, dans une conduite d'évacuation d'air (16) traversant le vérin et, dans l'autre position terminale, dans la chambre annulaire (13).

FIG.1

FIG.2

FIG. 3

FIG. 4